# EUROPÄISCHE PATENTSCHRIFT

(11) **EP 1 792 186 B1**
(45) Veröffentlichungstag und Bekanntmachung des Hinweises auf die Patenterteilung: **15.07.2009**
(21) Anmeldenummer: 05783050.7
(22) Anmeldetag: 13.09.2005
(51) Int. Cl.: G01N 33/574, C12Q 1/42, G01N 33/573

(54) **VERWENDUNGEN DER CARBAMOYLPHOSPHAT SYTHETASE 1 (CPS 1) ALS HUMORALER BIOMARKER FÜR DIE DIAGNOSE VON TUMORERKRANKUNGEN**
USES OF CARBAMOYL PHOSPHATE SYNTHETASE 1 (CPS 1) AS A HUMORAL BIOMARKER FOR THE DIAGNOSIS OF TUMOUR DISEASES
UTILISATIONS DE LA CARBAMOYLPHOSPHATE SYNTHETASE 1 (CPS 1) COMME BIOMARQUEUR HUMORAL POUR LE DIAGNOSTIC DE MALADIES TUMORALES

(30) Priorität: 21.09.2004 DE 102004045705
(43) Veröffentlichungstag der Anmeldung: 06.06.2007
(73) Patentinhaber: BioAssays GmbH, 16761 Henningsdorf (DE)
(72) Erfinder: BERGMANN, Andreas, 12351 Berlin (DE); STRUCK, Joachim, 13465 Berlin (DE)
(74) Vertreter: Andrae, Steffen
(86) Internationale Anmeldenummer: PCT/EP2005/009827
(87) Internationale Veröffentlichungsnummer: WO 2006/032392

(56) Entgegenhaltungen:
- EP-A- 1 403 282
- WO-A-99/63067
- WO-A-03/042661
- WO-A-03/089933
- LIU T-H ET AL: "CARBAMYL PHOSPHATE SYNTHETASE I A NOVEL MARKER FOR GASTRIC CARCINOMA" CHINESE MEDICAL JOURNAL (ENGLISH EDITION), Bd. 102, Nr. 8, 1989, Seiten 630-638, XP008057425 ISSN: 0366-6999 in der Anmeldung erwähnt
- LAWSON D ET AL: "Urea synthesis in Novikoff and Morris hepatomas" CANCER RESEARCH 1977, Bd. 37, Nr. 3, 1977, Seiten 850-856, XP008057503
- "Roche Lexikon Medizin" URBAN & SCHWARZENBERG , MÜNCHEN * Seite 855 *

## Beschreibung

Die vorliegende Erfindung betrifft neue Verwendungen des Enzyms Carbamoylphosphat Synthetase 1 (E.C. 6.3.4.16, nachfolgend stets abgekürzt CPS 1) und/oder physiologisch auftretender CPS 1-Fragmente mit CPS 1-Immunreaktivität als humoraler Biomarker für die medizinische Diagnostik zum Nachweis von Tumorerkrankungen (als humoraler Tumormarker), d.h. zur Diagnose von Tumorerkrankungen, durch Nachweis von CPS 1 bzw. CPS 1-Immunreaktivität in der Zirkulation, insbesondere in Serum oder Plasma von Patienten, bei denen eine Routineuntersuchung auf ein mögliches Vorhandensein von Tumoren durchgeführt wird, bei denen ein begründeter Verdacht auf Tumoren besteht, oder bei denen ein Tumor bereits nachgewiesen wurde und die überwacht bzw. einer Therapie unterzogen werden.

Der Begriff "Tumor" wird im Rahmen der vorliegenden Anmeldung als Oberbegriff bzw. Synonym für Neoplasmen, insbesondere bösartige Neoplasmen, verwendet, wie sie für Krebserkrankungen (Karzinome) typisch sind. An bösartigen Neubildungen (malignen Tumoren), d.h. Krebs, sind in einem Land wie Deutschland pro Jahr mehr als 300.000 Männer und Frauen erkrankt, wobei die Zahl der jährlich diagnostizierten neuen Krebsfälle sowie die Sterblichkeit erheblich sind. Maligne Tumoren können in nahezu jedem Gewebe bzw. Organ entstehen, und je nach betroffenem Organ unterscheidet man zahlreiche Krebserkrankungen, die sich im Hinblick auf ihre statistische Häufigkeit, Prognose und Therapierbarkeit erheblich voneinander unterscheiden können.

In den letzten Jahren wurde eine Vielzahl von Therapien entwickelt, die im Hinblick auf die Heilbarkeit zahlreicher Formen von Krebserkrankungen erhebliche Fortschritte mit sich gebracht haben. In allen Fällen gilt jedoch, dass die Heilungschancen von Krebserkrankungen stets besser werden, je früher die Krebserkrankung erkannt wird. Ganz besonders wichtig ist dabei eine Früherkennung von Krebs zu einem Zeitpunkt, zu dem noch keine oder noch keine signifikanten klinischen Symptome auftreten.

Zur möglichst frühen Erkennung von Tumoren in der klinischen Diagnose werden in biologischen Proben, insbesondere Blutproben und Körpersekreten, von untersuchten Patienten sog. "Tumormarker" bestimmt. Tumormarker sind Substanzen, die entweder von malignen Tumorzellen direkt gebildet werden oder die dadurch entstehen, dass Tumorzellen die Synthese des jeweiligen Markers in Nicht-Tumorzellen induzieren. Indem Tumormarker in erhöhter Konzentration in flüssigen biologischen Proben (humorale Tumormarker) bzw. im Gewebe lokalisiert (zelluläre Tumormarker) nachgewiesen werden, ermöglichen sie je nach den gegebenen Umständen des Einzelfalls eine Früherkennung maligner Tumoren in Risikogruppen, dienen sie der primären Tumordiagnostik, ermöglichen sie prognostische Aussagen und/oder eine Überwachung einer Tumortherapie und ggf. eine Früherkennung eines Tumorrezidivs. In der vorliegenden Anmeldung wird als Oberbegriff für alle genannten spezielleren Anwendungsmöglichkeiten (Indikationen) der Begriff "Diagnostik" verwendet. Ein Überblick über die derzeit in der klinischen Diagnostik genutzten Tumormarker findet sich in Lothar Thomas (Hrsg.), Labor und Diagnose, 5. erweiterte Auflage, Abschnitt 34, vgl. insbesondere Überblick 34.1 "Maligne Erkrankungen" auf den Seiten 956-961, sowie die Beiträge zu einzelnen klinisch genutzten Tumormarkern unter 34.2-34.17 auf den Seiten 916-1019.

Allen derzeit bestimmten Tumormarkern ist gemeinsam, dass die Sensitivität ihrer Bestimmung relativ beschränkt ist, und sie eine relativ hohe Organspezifität aufweisen. Die relativ hohe Organspezifität der gegenwärtig bestimmten Tumormarker hat einerseits den Vorteil, dass man bei ihrem Nachweis gleichzeitig eine Information über das Organ erhält, bei dem die ursächliche Krebserkrankung mit hoher Wahrscheinlichkeit aufgetreten ist. Eine hohe Spezifität ist jedoch insofern ein Nachteil, als Krebserkrankungen anderer Organe bei der Bestimmung organspezifischer Tumormarker nicht erkennbar werden bzw. für eine umfassende Krebsfrüherkennung die gleichzeitige Bestimmung zahlreicher verschiedener Tumormarker erforderlich ist. Die relativ geringe Sensivität der Bestimmung der bekannten Tumormarker (bei einer hohen Sensitivität einer Bestimmung werden die meisten oder alle Erkrankten richtig erkannt), die je nach Krebserkrankung und Tumormarker zwischen 20 und 80% liegt, hat zur Folge, dass die Gefahr einer Nichterkennung von Krebserkrankungen trotz der Bestimmung der an sich dafür geeigneten Tumormarker immer noch recht hoch ist.

Es besteht daher weiterhin ein Bedarf nach neuen humoralen Biomarkern, insbesondere humoralen Tumormarkern, die aufgrund einer charakteristischen eigenen Organspezifität bzw. Sensitivität eine verbesserte Diagnostik, insbesondere Krebsdiagnostik, entweder bei ihrer Bestimmung allein oder in Kombination mit der Bestimmung eines oder mehrerer anderer Biomarker/Tumormarker, ermöglichen.

Die vorliegende Erfindung betrifft die Identifizierung eines humoralen Biomarkers, der als neuer humoraler Tumormarker dienen kann, und alle sich aus dieser Identifizierung ergebenden mögliche Verwendungen auf dem Gebiet der Tumordiagnostik.

Die Ansprüche sollen diese Verwendungen bzw. in vitro-Diagnoseverfahren unter Bestimmung des neuen humoralen Tumormarkers patentrechtlich absichern.

Die vorliegende Erfindung beruht auf der überraschenden Feststellung, dass in der Zirkulation, d.h. insbesondere in Plasmen bzw. Seren, von Patienten, bei denen klinisch verschiedene Tumoren, insbesondere innerer Organe bzw. Weichteile, identifiziert worden waren, mit teilweise hervorragender Sensitivität deutlich erhöhte Konzentrationen des Enzyms Carbamoylphosphat Synthetase (CPS 1) bzw. eine starke CPS 1-Immunreaktivität nachweisbar waren, und zwar in einem klaren Unterschied zu gesunden Kontrollpersonen, was CPS 1 bzw. CPS 1-Immunreaktivität zu einem neuartigen humoralen Tumormarker macht.

Unter Verwendung eines Immunoassays, der im Zusammenhang mit der Sepsisdiagnose entwickelt worden war und der selektiv den Nachweis bzw. die Messung von CPS 1 bzw. CPS 1-Immunreaktivität in einem Serum oder Plasma eines humanen Patienten ermöglicht, wurde im Hause der Anmelderin festgestellt, dass auch in der Zirkulation von Patienten mit verschiedenen klinisch diagnostizierten Tumoren in stark erhöhten Konzentrationen CPS 1 bzw. eine starke CPS 1-Immunreaktivität gefunden werden können.

Im Rahmen der gleichen Untersuchung wurde ferner festgestellt, dass sich der gleiche Biomarker auch in der Zirkulation von Patienten mit chronisch entzündlichen Darmerkrankungen (M.C.; C.U.) deutlich erhöht findet, insbesondere bei den für diese Krankheiten typischen akuten Krankheitsschüben.

Für die medizinische Diagnostik spielten CPS 1 bzw. CPS 1-Fragmente mit CPS 1-Immunreaktivität traditionell keine praktische Rolle. Auf dem engeren einschlägigen Gebiet der Tumordiagnostik wurde CPS 1 noch nie als möglicher humoraler Tumormarker diskutiert.

Das Enzym CPS 1 ((E.C. 6.3.4.16) selbst ist jedoch seit langem gut bekannt. Es katalysiert die Umwandlung von Ammoniak, Bicarbonat und 2 ATP unter Bildung von Carbamoylphosphat im ersten Schritt des Harnstoffzyklus. Es spielt dabei auch eine Rolle bei der Biosynthese von Arginin, das seinerseits ein Substrat für die Biosynthese von NO, z.B. bei einem Endotoxin-Schock, ist (vgl. Shoko Tabuchi et al., Regulation of Genes for Inducible Nitric Oxide Synthase and Urea Cycle Enzymes in Rat Liver in Endotoxin Shock, Biochemical and Biophysical Research Communications 268, 221-224 (2000)). CPS 1 ist von dem cytosolischen Enzym CPS 2 (E.C. 6.3.5.5) zu unterscheiden, das ebenfalls eine Rolle im Harnstoffzyklus spielt, jedoch das Substrat Glutamin verarbeitet. Es ist bekannt, dass CPS 1 in Mitochondrien lokalisiert ist und im Lebergewebe in dieser Form in großen Mengen (es macht von 2-6% des Leber-Gesamtproteins aus) vorkommt. Seine Aminosäuresequenz und genetische Lokalisierung ist seit langem bekannt (vgl. Haraguchi Y. et al, Cloning and sequence of a cDNA encoding human carbamyl phosphate synthetase I: molecular analysis of hyperammonemia, Gene 1991, Nov. 1; 107 (2):335-340; vgl. auch die Veröffentlichung WO 03/089933 A1 der Anmelderin). Zu seiner physiologischen Rolle kann verwiesen werden auf Reviewartikel wie z.B. H.M.Holder et al., Carbamoyl phosphate synthetase: an amazing biochemical odyssey from substrate to product, CMLS, Cell.Mol.Life Sci. 56 (1999) 507-522 und die darin referierte Literatur, sowie die Einleitung der Veröffentlichung Mikiko Ozaki et al., Enzyme-Linked Immunosorbent Assay of Carbamoylphosphate Synthetase I: Plasma Enzyme in Rat Experimental Hepatitis and Its Clearance, Enzyme Protein 1994, 95:48:213-221.

Gemäß Shoko Tabuchi et al., loc.cit. wird in Rattenlebern bei einem künstlichen Endotoxin-Schock (LPS) keine Erhöhung des Enzyms (Proteins) beobachtet. Gemäß Li Yin et al., Participation of different cell types in the restitutive response of the rat liver to periportal injury induced by allyl alcohol, Journal of Hepatology 1999, 31:497-507, läßt sich bei einer Leberschädigung durch Allylakohol bei histologischen Untersuchungen nach drei Tagen in allen Hepatocyten eine Steigerung der CPS 1-Expression beobachten.

Es wurde ferner festgestellt, dass sich im Rattenmodell bei einer durch Verabreichung von Galactosamin experimentell erzeugten akuten Hepatitis im Rattenplasma eine stark erhöhte immunologische CPS 1-Aktivität findet (nachgewiesen mit einem ELISA mit anti-Ratten CPS 1 IgG aus Kaninchen), und zwar 24-48 h nach der Behandlung mit dem Hepatitis-auslösenden Galactosamin. Im Rattenplasma wurden während der akuten Hepatitis auch zunehmend CPS 1-Fragmente mit molaren Massen von ca. 140 und 125 kDa ohne sonstige nähere Charakterisierung (Sequenzzuordnung) erkennbar, während bei einer begleitenden Immunoblotting-Analyse in humanen Autopsie-proben keine CPS 1-Fragmente mit CPS 1-Immunreaktivität beobachtet werden konnten (Mikiko Ozaki et al., loc. cit.).

In einer Arbeit von Liu Tong-hua et al., Carbamoyl Phosphate Synthetase 1, A Novel Marker for Gastric Carcinoma, Chinese Medical Journal, 102(8):630-638, 1989 werden Ergebnisse von immunocytometrischen Untersuchungen an Gewebsproben aus verschiedenen operative entfernten Tumoren auf eine mögliche Anwesenheit von CPS 1 berichtet. Die Autoren fanden nur in Karzinomgewebe aus dem Magen Hinweise auf CPS 1-Immunreaktivität, dagegen nicht in anderen Tumorgeweben (Ösophagus, Dickdarm, Pankreas, Lunge, Brust, Ovarium, Niere, Prostata und Harnblase). Sie leiten daraus eine mögliche Eignung von CPS 1 als selektiver Gewebemarker, d.h. zellulärer Tumormarker, für Magenkrebs ab. Ein mögliches Auftreten von CPS 1 in der Zirkulation wird nicht diskutiert, und aus den beschriebenen Untersuchungen lassen sich keinerlei Folgerungen bezüglich einer evtl. Eignung von CPS 1 als humoraler Tumormarker ableiten.

In WO03/042661 wird ein Zusammenhang zwischen höherer CPS-1 Expression in Dickdarmgewebe und Dickdarm-Krebs gemacht. Die Eignung der CPS-1 als humoraler Marker ist nicht erwähnt.

Die in der vorliegenden Anmeldung beschriebenen Messungen stellen den ersten Bericht über ein Auftreten von CPS 1 in der Zirkulation von Tumorpatienten dar. Bisher wurde CPS 1, und auch nur in Untersuchungen der Anmelderin, lediglich in Serum bzw. Plasma von Sepsispatienten bestimmt (vgl. WO 03/089933 A1). Sepsispatienten, deren hochakute potentiell lebensbedrohende Erkrankung typischerweise in Intensivstationen überwacht und behandelt wird, stellen eine von Tumorpatienten, Tumor-Risikopatienten bzw. Patienten mit chronisch entzündlichen Darmerkrankungen, d.h. Patienten, die an einer sich über lange Zeiträume entwickelnden oder chronischen Erkrankung leiden, klar unterschiedene Patientenpopulation dar.

Bei der Bestimmung von CPS 1 bzw. CPS 1-Immunreaktivität als humoraler Biomarker/Tumormarker in Patientenseren gemäß der vorliegenden Erfindung kann grundsätzlich so vorgegangen werden, wie das in der Veröffentlichung WO 03/089933 A1 der Anmelderin im Zusammenhang mit der Bestimmung von CPS 1 als Sepsismarker beschrieben wurde. Das im Experimentellen Teil der vorliegenden Anmeldung beschriebene Bestimmmungsverfahren, das zur Prüfung von Seren bzw. Plasmen von Tumorpatienten auf die Anwesenheit von CPS 1 bzw. CPS 1-Immunreaktivität eingesetzt wurde, ist das gleiche Verfahren, das bereits in der o.g. Anmeldung WO 03/089933 A1 der Anmelderin beschrieben wurde.

Als "Verwendung" im Sinne der vorliegenden Anmeldung ist nicht nur die direkte immunologische Bestimmung von CPS 1 in in vitro Proben im Rahmen der Tumordiagnose, sondern auch eine Verwendung von CPS 1 bzw. CPS 1-Fragmenten, oder von Antikörpern zu deren selektiver Bestimmung, zur Herstellung von Assaykits, oder eine Verwendung zur Erzeugung von Assaykomponenten, z.B. von poly- oder monoklonalen Antikörpern, die z.B. in immobilisierter und/oder markierter Form in der Regel ebenfalls in Assaykits für die angegebenen Erkrankungen vorgesehen sind, oder von Standard- und Bezugssubstanzen.

Es ist zusätzlich ausdrücklich darauf hinzuweisen, dass bei der erfindungsgemäßen Bestimmung von CPS 1 oder CPS 1-Immunreaktivität je nach Assaydesign eine gleichzeitige Bestimmung sowohl von CPS 1 in Form des im wesentlichen vollständigen Moleküls als auch in Form von in der biologischen Flüssigkeit möglicherweise vorhandenen anderen, kürzeren Bruchstücken (physiologisch vorkommenden Teilpeptiden) der vollständigen CPS 1 erfolgen kann. Wenn in der vorliegenden Anmeldung von einer Bestimmung von "CPS 1-Immunreaktivität" gesprochen wird, soll das diesem messtechnischen Umstand Rechnung tragen, damit eine unsachgemäße einengende Auslegung der Lehre der vorliegenden Erfindung vermieden wird.

An Stelle der Bestimmung von CPS 1 oder CPS 1-Immunreaktivität soll für diagnostische Zwecke die CPS 1-Bestimmung ggf. auch indirekt als Bestimmung einer Enzymaktivität, die der CPS 1-Aktivität oder der Restaktivität der CPS 1-Fragmente im Blut entspricht, erfolgen können. Da CPS 1 bei Gesunden in der Zirkulation nicht auftritt, kann eine messbare CPS 1 Enzymaktivität im Blut eines Patienten ein diagnostisch signifikantes Indiz für eine ernsthafte Störung der gesundheitlichen Unversehrtheit des Patienten sein. Es sei an dieser Stelle auch noch darauf hingewiesen, dass die Aktivität eines Enzyms, das normalerweise im Zellinneren lokalisiert ist und nur dort seine funktionsgerechte Wirkung entfaltet, in der Zirkulation per se negativ zu bewerten ist und daher auch als solche zu einer Verschärfung des Krankheitszustands beitragen kann.

In Plasma bzw. Serum nachweisbare CPS 1 bzw. CPS 1-Immunreaktivität eignen sich auf der Basis der nachfolgend gezeigten Ergebnisse als spezifische Markerpeptide (Tumormarker) zum diagnostischen Nachweis von Tumoren (Neoplasmen).

Es ist ferner vorgesehen, die Bestimmung von CPS 1 und/oder CPS 1-Fragmenten als Prognosemarker und Marker für die Überwachung des Krankheitsverlaufs von Tumorerkrankungen im Rahmen einer Kombinationsmessung mit anderen Markern durchzuführen.

Die eigentliche CPS 1-Bestimmung kann auf irgendeine geeignete, an sich bekannte Weise erfolgen, wobei Immunoassays eines geeigneten Assaydesigns bevorzugt sind.

Die Verfahren zur Bestimmung von CPS 1-Immunreaktivität in einer biologischen Probe können beliebige bekannte Verfahren der Immundiagnostik sein, die zum Nachweis und zur Messung von Antigenen angewandt werden. Vorzugsweise wird CPS 1 mit Hilfe eines Ligandenbindungsassays bestimmt, bei dem man zur Bindung und Markierung geeignete spezifische Antikörper in immobilisierter Form bzw. markierter bzw. markierbarer Form verwendet.

Auch kompetitive Assayformate können besondere Vorteile bieten. Vorzugsweise wird dabei nicht mit einer Enyzmmarkierung gearbeitet, sondern es wird eine andere Markierung gewählt, z.B. eine Markierung für eine Chemilumineszenz-Nachweisreaktion, z.B. ein Akridiniumester. Selbstverständlich ist vorzugsweise ein solcher Assay zur CPS 1-Bestimmung zu verwenden, der die benötigte hohe Sensitivität im Bereich der vorkommenden CPS 1-Konzentrationen gewährleistet und eine Separierung der Messignale vom Assay-Hintergrund ermöglicht.

Das Bestimmungsverfahren kann an die Chip-Technologie angepaßt sein oder als Schnelltest (Point-of-Care-Test) ausgestaltet werden.

Bei einer bevorzugten Ausführungsform wird die immundiagnostische Bestimmung als heterogener Sandwich-Immunoassay durchgeführt, bei dem einer der Antikörper an eine beliebige Festphase, beispielsweise die Wände beschichteter Teströhrchen (z.B. aus Polystyrol; "Coated Tubes"; CT) oder an Mikrotiterplatten, zum Beispiel aus Polystyrol, oder an Partikel, beispielsweise Magnetpartikel immobilisiert ist, während der andere Antikörper einen Rest trägt, der ein direkt nachweisbares Label darstellt oder eine selektive Verknüpfung mit einem Label ermöglicht und der Detektion der gebildeten Sandwich-Strukturen dient. Auch eine zeitlich verzögerte bzw. nachträgliche Immobilisierung unter Verwendung geeigneter Festphasen ist möglich.

Grundsätzlich können alle in Assays der beschriebenen Art verwendbaren Markierungstechniken angewandt werden, zu denen Markierungen mit Radioisotopen, Enzymen, Fluoreszenz-, Chemolumineszenz- öder Biolumineszenz-Labeln und direkt optisch detektierbaren Farbmarkierungen, wie beispielsweise Goldatomen und Farbstoffteilchen, wie sie insbesondere für sog. Point-of-Care (POC) oder Schnelltests verwendet werden, gehören. Die beiden Antikörper können auch im Falle heterogener Sandwich-Immunoassays Teile eines Nachweissystems der nachfolgend im Zusammenhang mit homogenen Assays beschriebenen Art aufweisen.

Das erfindungsgemäße Verfahren kann ferner als homogenes Verfahren ausgestaltet werden, bei dem die aus den beiden Antikörpern und dem nachzuweisenden CPS 1 gebildeten Sandwichkomplexe in der flüssigen Phase suspendiert bleiben. In einem solchen Fall ist es bevorzugt, beide Antikörper mit Teilen eines Nachweissystems zu markieren, das dann, wenn beide Antikörper in einen einzigen Sandwich integriert werden, eine Signalerzeugung oder Signalauslösung ermöglicht. Derartige Techniken sind insbesondere als Fluoreszenzverstärkungs- oder Fluoreszenzlöschungs-Nachweisverfahren ausgestaltbar. Ein besonderes bevorzugtes derartiges Verfahren betrifft die Verwendung von paarweise einzusetzenden Nachweisreagenzien, wie sie beispielsweise beschrieben sind in US-A-4 822 733, EP-B1-180 492 oder EP-B1-539 477 und dem darin zitierten Stand der Technik. Sie ermöglichen eine Messung, die selektiv nur Reaktionsprodukte erfaßt, die beide Markierungskomponenten in einem einzigen Immunkomplex enthalten, direkt in der Reaktionsmischung. Als Beispiel ist auf die unter den Marken TRACE^{®} (Time Resolved Amplified Cryptate Emission) bzw. KRYPTOR^{®} angebotene Technologie zu verweisen, die die Lehren der o.g. Anmeldungen umsetzt.

Der Inhalt der genannten älteren Anmeldung (WO 03/089933 A1) der Anmelderin ist durch die ausdrückliche Bezugnahme auf diese Anmeldungen als Teil der Offenbarung der vorliegenden Anmeldung anzusehen.

Nachfolgend werden die Bestimmung von CPS 1 und die bei ihrer Bestimmung in Plasmen von Tumorpatienten und Patienten mit M.C. oder C.U. gewonnenen Befunde noch näher erläutert, wobei auf zwei Figuren Bezug genommen wird.

Darin zeigen:
- Figur 1: die Ergebnisse der Messung der CPS 1-Immunreakti- vität in Plasmen von gesunden Normalpersonen und von Patienten mit verschiedenen, in der Figur angegebenen klinisch diagnostizierten Tumorerkran- kungen mit dem im experimentellen Teil genauer beschriebenen Immunoassay, wobei die gestrichelte Linie die untere Nachweisgrenze des Tests (0,5 ng/ml) anzeigt.
- Figur 2: entsprechende Ergebnisse in Plasmen von Patienten, die einen akuten Schub ihrer chronisch-entzündli- chen Darmerkrankung (M.C. oder C.U.) erleiden.

### EXPERIMENTELLER TEIL

### CPS 1-Immunreaktivitäts-Bestimmungen in humanen Plasmen von gesunden Normalpersonen und Tumorpatienten

### 1. Material und Methoden

### 1.1. Peptidsynthesen

Abgeleitet von der bekannten Aminosäuresequenz des humanen CPS 1 wurden zwei Bereiche ausgewählt (Pos. 184-199: Peptidbereich 1; SEQ ID NO:1; Pos. 245-257: Peptidbereich 2; SEQ ID NO:2). Jeweils ergänzt um einen N-terminalen Cystein-Rest wurden beide Bereiche nach Standardverfahren als lösliche Peptide chemisch synthetisiert, gereinigt, mittels Massenspektrometrie und Reversed Phase HPLC qualitätskontrolliert und in Aliquots lyophilisiert (Firma JERINI AG, Berlin, Deutschland). Die Aminosäuresequenzen der Peptide lauten:
Peptid PCEN17: CEFEGQPVDFVDPNKQN SEQ ID NO:1
Peptid PCVD14: CVPWNHDFTKMEYD SEQ ID NO:2

Rekombinantes Standardmaterial wurde von der Fima InVivo GmbH (Hennigsdorf, Deutschland) bezogen. Es handelte sich um einen kruden Zellextrakt eines E. coli-Stamms, der den rekombinanten N-terminalen Bereich von humaner CPS 1, ergänzt um einen N-terminalen Strep-tag, exprimierte. Dem Extrakt wurde eine arbiträre Konzentration an CPS 1 zugeschrieben.

### 1.2. Konjugation und Immunisierung

Mittels MBS (m-Maleimidobenzoyl-N-hydroxysuccinimid Ester) wurden die o.g. Peptide PCEN17 und PCVD14 an das Trägerprotein KLH (Keyhole limpet hemocyanin) konjugiert (s. Arbeitsanleitung "NHS-Esters-Maleimide Crosslinkers" Firma PIERCE, Rockford, IL, USA). Mit diesen Konjugaten wurden Schafe nach folgendem Schema immunisiert: Jedes Schaf erhielt initial 100 µg Konjugat (Massenangabe bezogen auf den Peptid-Anteil des Konjugats) und anschließend 4-wöchentlich je 50 µg Konjugat (Massenangabe bezogen auf den Peptid-Anteil des Konjugats). Beginnend mit dem vierten Monat nach Beginn der Immunisierung wurden 4-wöchentlich je Schaf 700 ml Blut abgenommen und daraus durch Zentrifugation Antiserum gewonnen. Konjugationen, Immunisierungen und Gewinnung von Antiseren wurden von der Firma MicroPharm, Carmarthenshire, UK, durchgeführt.

### 1.3. Reinigung der Antikörper

In einem 1-Schritt Verfahren wurden aus den Antiseren, die beginnend mit dem vierten Monat nach der Immuniserung gewonnen worden waren, die Peptid-spezifischen Antikörper präpariert. ,

Dazu wurden zunächst die Peptide PCEN17 und PCVD14 an SulfoLink Gel gekoppelt (s. Arbeitsanleitung "SulfoLink Kit" Firma PIERCE, Rockford, IL, USA). Dabei wurden zur Kopplung je 5 mg Peptid pro 5 ml Gel angeboten.

Die Affinitätsreinigung von Peptid-spezifischen Antikörpern aus Schaf Antiseren gegen beide Peptide wurde wie folgt durchgeführt:
Die Peptid-Säulen wurden zunächst drei mal im Wechsel mit je 10 ml Elutionspuffer (50 mM Citronensäure, pH 2.2) und Bindungspuffer (100 mM Natriumphosphat, 0.1% Tween, pH 6.8) gewaschen. 100 ml der Antiseren wurden über 0,2 µm filtriert und mit dem vorhandenen Säulenmaterial versetzt. Dazu wurde das Gel quantitativ mit 10 ml Bindungspuffer aus der Säule gespült. Die Inkubation erfolgte über Nacht bei Raumtemperatur unter Schwenken. Die Ansätze wurden quantitativ in Leersäulen (NAP 25, Pharmacia, entleert) überführt . Die Durchläufe wurden verworfen. Anschließend wurde mit 250 ml Bindungspuffer proteinfrei (Proteingehalt des Wascheluats < 0,02 A280 nm) gewaschen. Auf die gewaschene Säulen wurde Elutionspuffer gegeben, und es wurden Fraktionen à 1 ml gesammelt. Von jeder Fraktion wurde der Proteingehalt mittels der BCA-Methode (s. Arbeitsanleitung Firma PIERCE, Rockford, IL, USA) bestimmt. Fraktionen mit Proteinkonzentrationen > 0.8 mg/ml wurden gepoolt. Nach Proteinbestimmung der Pools mittels der BCA-Methode ergaben sich Ausbeuten von 27 mg für den anti-PCEN17 Antikörper und 33 mg für den anti-PCVD14 Antikörper.

### 1.4. Markierung

Über eine NAP-5 Gelfiltrationssäule (Pharmacia) wurden 500 µl des gereinigten anti-PCEN17 Antikörpers (s.o.) in 1 ml 100 mM Kalium-Phosphatpuffer (pH 8,0) nach Arbeitsanleitung umgepuffert. Die Proteinkonzentationsbestimmung der Antikörperlösung ergab einen Wert von 1,5 mg/ml.

Zur Chemilumineszenzmarkierung des Antikörpers wurden 67 µl der Antikörperlösung mit 10 µl MA70-Akridinium-NHS-Ester (1 mg/ml; Firma HOECHST Behring) versetzt und 15 Minuten bei Raumtemperatur inkubiert. Dann wurden 423 µl 1 M Glycin zugesetzt und weitere 10 Minuten inkubiert. Anschließend wurde der Markierungsansatz über eine NAP-5 Gelfiltrationssäule (Pharmacia) in 1 ml Laufmittel A (50 mM Kaliumphosphat, 100 mM NaCl, pH 7.4) nach Arbeitsanleitung umgepuffert und dabei von niedermolekularen Bestandteilen befreit. Zur Abtrennung letzter Reste nicht an Antikörper gebundenen Labels wurde eine Gelfiltrations-HPLC durchgeführt (Säule: Waters Protein Pak SW300). Die Probe wurde aufgetragen und bei einer Flußrate von 1 ml/min mit Laufmittel A chromatographiert. Mit einem Duchflußphotometer wurden die Wellenlängen 280 nm und 368 nm gemessen. Das Absorptionsverhältnis 368 nm/280 nm als Maß für den Markierungsgrad des Antikörpers betrug am Peak 0.10. Die monomeren Antikörper enthaltenden Fraktionen (Retentionszeit 8-10 min) wurden gesammelt und in 3 ml 100 mM Natriumphosphat, 150 mM NaCl, 5% Bovines Serum Albumin, 0.1% Natrium Azid, pH 7.4, gesammelt.

### 1.5. Kopplung

Bestrahlte 5 ml Polystyrolröhrchen (Firma Greiner) wurden mit gereinigtem anti-PCVD14 Antikörper wie folgt beschichtet: Der Antikörper wurde in 50 mM Tris, 100 mM NaCl, pH 7,8 zu einer Konzentration von 6.6 µg/ml verdünnt. In jedes Röhrchen wurden 300 µl dieser Lösung pipettiert. Die Röhrchen wurden 20 Stunden bei 22°C inkubiert. Die Lösung wurde abgesaugt. Dann wurde jedes Röhrchen mit 4.2 ml 10 mM Natriumphosphat, 2% Karion FP, 0.3% Bovines Serum Albumin, pH 6.5 befüllt. Nach 20 Stunden wurde die Lösung abgesaugt. Schließlich wurden die Röhrchen in einem Vakuumtrockner getrocknet.

### 2. Durchführung und Auswertung des Immunoassays

### 2.1. Assaygestaltung

Es wurde ein Assaypuffer folgender Zusammensetzung hergestellt:
100 mM Natriumphosphat, 150 mM NaCl, 5% Bovines Serum Albumin, 0.1% unspez. Schaf IgG, 0.1% Natrium Azid, pH 7.4

Als Standardmaterial diente in E. coli exprimierte rekombinante humane CPS 1 in Form eines kruden E. coli-Extrakts, enthaltend das gesamte lösliche intrazelluläre Protein. Dieser Extrakt wurde seriell in Pferdenormalserum (Firma SIGMA) verdünnt. Den so hergestellten Standards wurden arbiträre Konzentrationen gemäß ihrer Verdünnung zugeschrieben.

### 2.2. Messung von EDTA-Plasmen von augenscheinlich Gesunden und von Patienten mit verschiedenen Neoplasmen (Tumoren)

### Testseren:

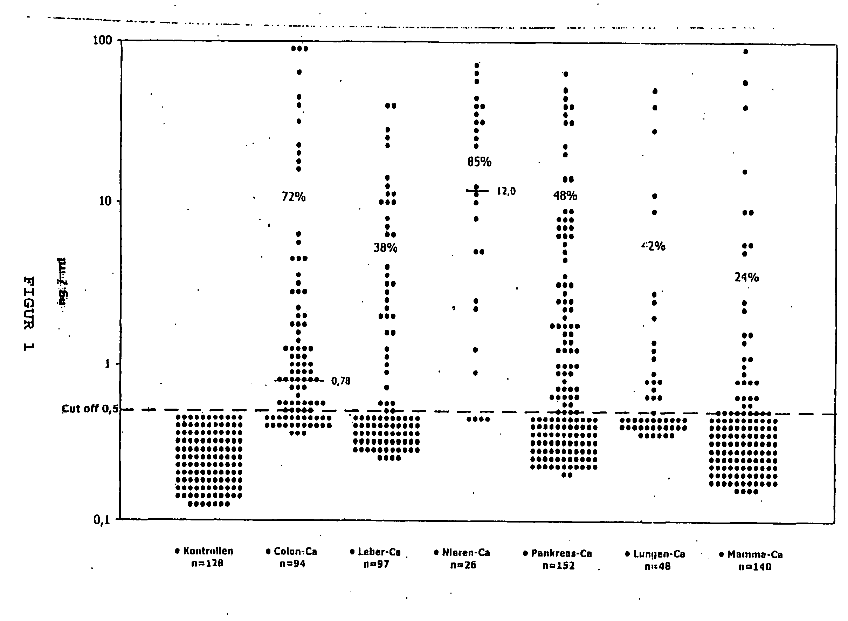

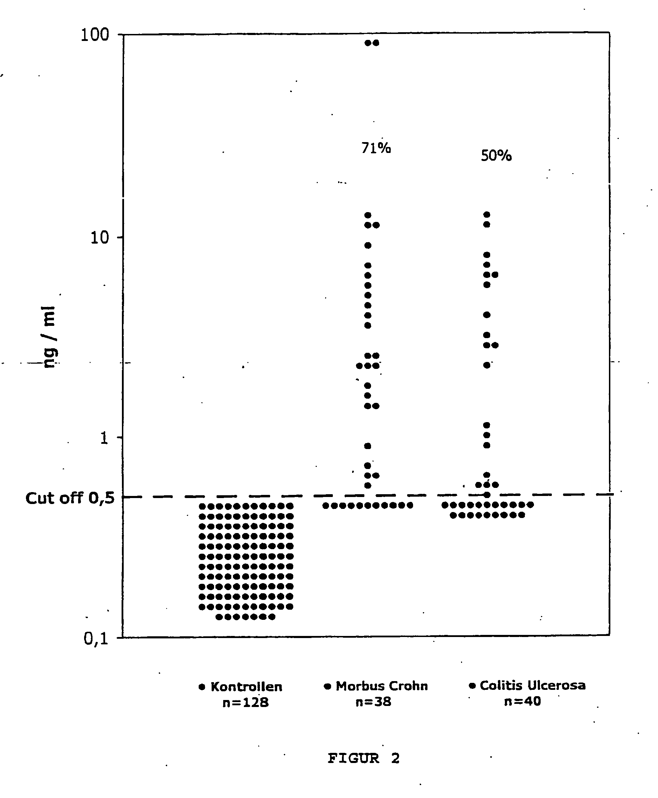

a. Als Testseren für die CPS 1-Bestimmungen dienten einmal 557 Plasmen von verschiedenen Patienten mit klinisch diagnostizierten Tumoren verschiedener Organe/Gewebe. Zu jedem Testplasma existierte eine genaue klinische Dokumentation, die eine Aufschlüsselung der in die Messung eingesetzten Plasmen der Patienten nach der bei ihnen festgestellten Tumorart ermöglichte.
   Genauer wurden vermessen: 94 Plasmen von Patienten mit Dickdarm-Krebs (in Figur 1: Colon-Ca), 97 Plasmen von Patienten mit Leber-Krebs (Leber-Ca), 26 Plasmen von Patienten mit Nieren-Krebs (Nieren-Ca), 152 plasmon von Patienten mit Pankreas-Krebs (Pankreas-Ca), 48 Plasmen von Patienten mit Lungen-Krebs (Lungen-Ca) und 140 Plasmen von Patientn mit Brustkrebs (Mamma-Ca).
   Als Kontrollseren dienten 128 Seren von augenscheinlich Gesunden.
b. Parallel dazu wurden auch Seren von insgesamt 78 Patienten vermessen, bei denen eine Behandlung eines akuten Schubs von Morbus Crohn (M.C.) oder Colitis ulcerosa (C.U.) durchgeführt wurde (Figur 2).

In die o.g. Teströhrchen wurden je 50 µl Standard bzw. Probe sowie 200 µl Assaypuffer pipettiert. Es wurde 18 Stunden bei 22°C unter Schütteln inkubiert. Dann wurde 4 x mit je 1 ml Waschlösung (0.1% Tween 20) pro Röhrchen gewaschen. Dann wurde in jedes Röhrchen 200 µl Assaypuffer, enthaltend 0.5 Millionen RLU des MA70-markierten Tracer-Antikörpers, pipettiert. Es wurde zwei Stunden bei 22°C unter Schütteln inkubiert. Dann wurde 4 x mit je 1 ml Waschlösung (0.1% Tween 20) pro Röhrchen gewaschen, abtropfen gelassen und die am Röhrchen gebundene Chemilumineszenz in einem Luminometer (Firma BERTHOLD, LB952T; Basisreagenzien BRAHMS AG) vermessen.

Unter Verwendung der Software MultiCalc (Spline Fit) wurde die Konzentration an CPS 1-Immunreaktivität abgelesen. Die Ergebnisse für Tumorpatienten sind in Figur 1 gezeigt, die Ergebnisse für Patienten mit chronisch entzündlichen Darmerkrankungen (M.C.; C.U.) in Figur 2. Es ist in jedem Fall eine eindeutige Unterscheidung von Gesunden, bei denen keine über der Nachweisgrenze (0,5 ng/ml) liegenden Konzentrationen von CPS 1 gefunden wurden, und Patienten zu erkennen, wobei die Sensitivität des Nachweises von CPS 1 für verschiedene Tumorarten durchaus verschieden war.

Und zwar wurden für die verschiedenen Krebsarten die folgenden Sensitivitäten ermittelt (vgl. Fig.1):

| | |
|---|---|
| Dickdarm-Krebs | 72% |
| Leber-Krebs | 38% |
| Nieren-Krebs | 85% |
| Pankreas-Krebs | 48% |
| Lungen-Kebs | 42% |
| Brust-Krebs | 24%. |

Die Sensitivität bei entzündlichen Darmerkrankungen (Morbus Crohn/Colitis ulcerosa), lag für M.C. bei 71% und für C.U. bei 50%.

Mittels des beschriebenen Sandwichimmunoassays wurde somit gezeigt, daß Plasmen von Tumorpatienten und Patienten in einer akuten Phase einer chronisch entzündlichen Darmerkrankung stark erhöhte Konzentrationen von CPS 1-Immunreaktivität aufweisen können, während in Plasmen von Gesunden CPS 1 nicht zu detektieren war. Es zeigte sich für verschiedene Tumorpatienten eine massive Erhöhung der CPS 1-Immunreaktivität in Plasma. Diese ist in keinen klaren logischen Zusammenhang zum vorbekannten Auftreten von CPS 1 bei Sepsis zu bringen. Es ist bekannt, daß bei der Sepsis eine Schädigung der Mitochondrien eintritt (Crouser ED et al., Endotoxin-induced mitochondrial damage correlates with impaired respiratory activity; Crit Care Med. 2002 Feb; 30(2):276-84). Eine derartige Schädigung in Verbindung mit Nekrose oder Apoptose könnte Ursache des Übertritts von CPS 1 aus der mitochondrialen Matrix in die Blutzirkulation bei Sepsis sein. Diese Annahme zur Erklärung des Auftretens von CPS 1 bei Sepsis liefert jedoch keine einfache plausible Erklärung für das Auftreten in Seren bzw. Plasmen von Tumorpatienten, das aufgrund der Erkenntnisse des Standes der Technik nicht vorhersagbar war.

Die der vorliegenden Erfindung zugrunde liegenden Erkenntnisse über das Auftreten erheblicher Konzentrationen von CPS 1 in der Zirkulation von Krebspatienten und M.C. bzw. C.U.-Patienten lassen es als möglich erscheinen, dass CPS 1 auch in gelöster Form wenigstens Teile seiner Enzymreaktivität behalten hat und zur Verschärfung der Erkrankung und/oder zu bestimmten unerwünschten Krankheitsfolgen beiträgt. Daraus ergibt sich, dass an sich bekannte Substanzen, die die Expression oder die enzymatische Wirkung von CPS 1 inhibieren, geeignet sein können, das Gesamt-Krankheitsgeschehen positiv zu beeinflussen. Derartige Substanzen sind z.B. beschrieben in J Steroid Biochem Mol Bio 1991 May; 38(5):-599-609; J Biol Chem 1977 May 25; 252(10):3558-60; J Biol Chem 1984 Jan 10; 259(1):323-31 und J Biol Chem 1981 Nov 10; 256(21):11160-5; J Biol Chem 1981 Apr 10; 256(7):3443-6. Zu ihnen gehören insbesondere Ca-Ionen und andere Metallionen und Substanzen vom Steroidtyp. Zu ihnen sind ferner auch Antikörper oder andere spezifische Binder zu zählen, die als CPS 1-Hemmer oder -Inhibitoren durch Bindung an CPS 1 die Wirkung von CPS 1 in der Zirkulation aufheben oder abschwächen können.

Demgemäß stellt die Verabreichung von derartigen CPS 1-Inhibitoren an Patienten, bei denen im Blut CPS 1 nachweisbar ist, ein Mittel dar, das Krankheitsgeschehen therapeutisch zu beeinflussen und den Zustand und/oder das Wohlbefinden eines Krebspatienten signifikant zu verbessern.

## Patentansprüche

1. Verwendung der Carbamoylphosphat Synthetase (CPS 1) und/oder ihrer Fragmente mit CPS 1-Immunreaktivität als humoraler Biomarker für die in vitro Diagnose, Verlaufssowie Therapiekontrolle von Tumorerkrankungen.

2. In vitro-Verfahren zur Früherkennung und Erkennung, für die Verlaufsprognose und die Beurteilung des Schweregrads sowie zur therapiebegleitenden Verlaufsbeurteilung von Tumorerkrankungen, **dadurch gekennzeichnet, dass** man in einer Serum- oder Plasmaprobe eines Patienten die Anwesenheit und/oder Menge von CPS 1 und/oder ihrer physiologisch vorkommenden Fragmente mit CPS 1-Immunreaktivität bestimmt und aus dem Nachweis und/oder der Menge von CPS 1 bzw. CPS 1-Immunreaktivität Schlüsse hinsichtlich des Vorliegens, des zu erwartenden Verlaufs, des Schweregrads oder des Erfolgs eines Therapie einer Tumorerkrankung zieht.

3. Verfahren nach Anspruch 2, **dadurch gekennzeichnet, dass** die zur Bestimmung genutzten CPS 1-Sequenzen Sequenzen aus einem die Aminosäuren 1 bis ca. 630 der vollständigen CPS 1-Sequenz umfassenden N-terminalen Teil der CPS 1 sind.

4. Verfahren nach einem der Ansprüche 2 oder 3, **dadurch gekennzeichnet, dass** es ein immundiagnostisches Bestimmungsverfahren ist.

5. Verfahren nach einem der Ansprüche 2 oder 3, **dadurch gekennzeichnet, dass** es ein Verfahren zur Bestimmung der CPS 1-Enzymaktivität oder einer CPS 1-Restenzymaktivität in einer Vollblut-, Serum - oder Plasamprobe eines Krebspatienten ist.

6. Verfahren nach einem der Ansprüche 2 bis 5, **dadurch gekennzeichnet, dass** es zum Nachweis eines Tumors durchgeführt wird, der ausgewählt ist aus einer Gruppe, die Dickdarm-Karzinome, Leber-Karzinome, Nieren-Karzinome, Pankreas-Karzinome, Lungen-Karzinome und Mamma-Karzinome und Kombinationen davon umfaßt.

## Claims

1. Use of carbamoyl phosphate synthetase (CPS 1) and/or its fragments having CPS 1 immunoreactivity as a humoral biomarker for the *in vitro* diagnosis and monitoring of progress and treatment of tumour diseases.

2. *In vitro* method for the early diagnosis and detection, for progress prognosis and the evaluation of severity and for treatment-accompanying progress evaluation of tumour diseases, **characterized in that** the presence and/or the amount of CPS 1 and/or its physiologically occurring fragments having CPS 1 immunoreactivity are determined in a serum or plasma sample of a patient and conclusions are drawn from the detection and/or the amount of CPS 1 or CPS 1 immunoreactivity concerning the presence, the expected progress, the severity or the success of the treatment of a tumour disease.

3. Method according to Claim 2, **characterized in that** the CPS 1 sequences used for the determination are sequences from an N-terminal part of CPS 1, which part comprises the amino acids 1 to about 630 of the complete CPS 1 sequence.

4. Method according to either of Claims 2 and 3, **characterized in that** it is an immunodiagnostic assay method.

5. Method according to either of Claims 2 and 3, **characterized in that** it is a method for determining the CPS 1 enzyme activity or a CPS 1 residual enzyme activity a whole blood, serum or plasma sample of a cancer patient.

6. Method according to any of Claims 2 to 5, **characterized in that** it is carried out for detecting a tumour which is selected from a group comprising carcinomas of the large intestine, liver carcinomas, kidney carcinomas, pancreatic carcinomas, lung carcinomas and breast carcinomas and combinations thereof.

## Revendications

1. Utilisation de la synthétase de carbamoylphosphate (CPS 1) et / ou de ses fragments avec immunoréactivité CPS 1, en tant que bio-marqueur pour le diagnostic in vitro, le contrôle de l'évolution, ainsi que le contrôle de la thérapie de maladies tumorales.

2. Procédé in vitro pour la détection précoce et l'identification, pour le pronostic de l'évolution et l'évaluation du degré de gravité, ainsi que pour l'évaluation de l'évolution concomitante de la thérapie de maladies tumorales, **caractérisé en ce que,** dans un échantillon de sérum et / ou de plasma d'un patient, on détermine la présence et ou la quantité de CPS 1 et / ou de ses fragments, se présentant au niveau physiologique, avec immunoréactivité CPS 1, et que l'on tire de la présence et / ou de la quantité de CPS 1, respectivement de l'immunoréactivité CPS 1 des conclusions en ce qui concerne la présence, l'évolution prévisible, le degré de gravité ou le succès d'une thérapie de la maladie tumorale.

3. Procédé selon la revendication 2, **caractérisé en ce que,** les séquences CPS 1 utilisées pour la détermination, sont des séquences d'une partie de la CPS 1 terminal N comprenant les acides aminés 1 jusqu'à environ 630 de la séquence CPS 1 complète.

4. Procédé selon revendication 2 ou 3, **caractérisé en ce que** ce procédé est un procédé de détermination immunodiagnostique.

5. Procédé selon revendication 2 ou 3, **caractérisé en ce que** ce procédé est un procédé pour la détermination de l'activité enzymatique CPS 1 ou d'une activité enzymatique résiduelle CPS 1 dans un échantillon de sang complet, de sérum ou de plasma d'un patient cancéreux.

6. Procédé selon l'une des revendications 2 à 5, **caractérisé en ce qu'**il est utilisé pour la détection de la présence d'une tumeur, qui est sélectionnée dans un groupe comprenant les carcinomes du côlon, les carcinomes du foie, les carcinomes des reins, les carcinomes du pancréas, les carcinomes des poumons, les carcinomes du sein et les combinaisons de ceux-ci.


### SEQUENCE LISTING

<110> B.R.A.H.M.S Aktiengesellschaft
<120> Verwendung der Carbamoylphosphat Synthetase 1 (CPS 1) als humoraler Biomarker für die Diagnose von Tumorerkrankungen und chronisch entzündlichen Darmerkrankungen
<130> 4467 PCT
<150> DE 102004045705.0
   <151> 2004-09-21
<160> 2
<170> PatentIn version 3.2
<210> 1
   <211> 17
   <212> PRT
   <213> Synthetic Sequence
<400> 1
   Cys Glu Phe Glu Gly Gln Pro Val Asp Phe Val Asp Pro Asn Lys Gln
   1               5                   10                  15
   Asn
<210> 2
   <211> 14
   <212> PRT
   <213> Synthetic Sequence
<400> 2
   Cys Val Pro Trp Asn His Asp Phe Thr Lys Met Glu Tyr Asp
   1 5 10